# EUROPEAN PATENT SPECIFICATION

(11) **EP 1 489 994 B1**
(45) Date of publication and mention of the grant of the patent: **04.06.2008**
(21) Application number: 03746110.0
(22) Date of filing: 02.04.2003
(51) Int. Cl.: A61F 2/01, A61M 25/01

(54) **GUIDE WIRE APPARATUS FOR PREVENTION OF DISTAL ATHEROEMBOLIZATION**
FÜHRUNGSDRAHTAPPARAT ZUR VERHINDERUNG DISTALER ATHEROEMBOLISATION
APPAREIL DE FIL GUIDE POUR LA PREVENTION D'UNE ATHEROEMBOLISATION DISTALE

(30) Priority: 04.04.2002 US 116238
(43) Date of publication of application: 29.12.2004
(73) Proprietor: Medtronic Vascular, Inc., Santa Rosa, CA 95403 (US)
(72) Inventor: DOUK, Nareak, Lowell, MA 01850 (US); RAFIEE, Nasser, Andover, MA 01810 (US); NOONE, Michael, S., Londonderry, NH 03053 (US)
(74) Representative: Bauer, Friedrich
(86) International application number: PCT/US2003/010097
(87) International publication number: WO 2003/084436

(56) References cited:
- WO-A-01/12104
- WO-A-01/45592
- WO-A-99/22673
- US-B1- 6 355 051

## Description

### FIELD OF THE INVENTION

The present invention relates generally to intraluminal devices for trapping particulate in the vessels of a patient. More particularly, the invention relates to protection elements for trapping atheroemboli in a blood vessel during an interventional vascular treatment and then removing the trapped atheroemboli from the patient after completion of the treatment. Furthermore, the invention concerns a filter or an occluder mounted on a guide wire that can also be used to direct an interventional catheter to a treatment site within a patient.

### BACKGROUND OF THE INVENTION

A variety of treatments exists for dilating or removing atherosclerotic plaque in blood vessels. The use of an angioplasty balloon catheter is common in the art as a minimally invasive treatment to enlarge a stenotic or diseased blood vessel. When applied to the vessels of the heart, this treatment is known as percutaneous transluminal coronary angioplasty, or PTCA. To provide radial support to the treated vessel in order to prolong the positive effects of PTCA, a stent may be implanted in conjunction with the procedure.

Thrombectomy is a minimally invasive technique for removal of an entire thrombosis or a sufficient portion of the thrombosis to enlarge the stenotic or diseased blood vessel and may be accomplished instead of a PTCA procedure. Atherectomy is another well-known minimally invasive procedure that mechanically cuts or abrades a stenosis within the diseased portion of the vessel. Alternatively, ablation therapies use laser or RF signals to superheat or vaporize the thrombus within the vessel. Atheroemboli loosened during such procedures may be removed from the patient through the catheter.

During each of these procedures, there is a risk that atheroemboli dislodged by the procedure will migrate through the circulatory system and cause ischaemic events, such as infarction or stroke. Thus, practitioners have approached prevention of escaped atheroemboli through use of occlusion devices, filters, lysing, and aspiration techniques. For example, it is known to remove the atheroembolic material by suction through an aspiration lumen in the treatment catheter or by capturing atheroemboli in a filter or occlusion device positioned distal of the treatment area.

WO 99/22673 A and WO 01/45592 A disclose an apparatus for prevention of distal atheroembolization according to the features of the preamble of claim 1. These known apparatus comprise a tubular protection element the deployment of which is assisted by a spring.

### SUMMARY OF THE INVENTION

The problem addressed by the invention is to provide an apparatus for prevention of distal atheroembolization during percutaneous catheter invention procedures, such as angioplasty or stent deployment, the apparatus assuring a particularly reliable collapsing of a protection element.

This problem is solved by an apparatus comprising the features of claim 1. The dependent claims show advantageous embodiments of the inventive apparatus.

According to the characterizing portion of claim 1, the apparatus further comprises a coiled compression spring disposed within the tubular protection element and having distal and proximal ends coupled to the distal and proximal ends of the protection element, respectively, such that the compression spring urges the tubular protection element to remain in the closed configuration, the compression spring being sized and shaped to fit slidably over a core wire.

The expandable protection element, such as a filter or an occluder, is mountable adjacent the distal end of a core wire, which can guide a therapeutic catheter. As distinguished from filter guide wires, occluder guide wires are typically used to temporarily obstruct fluid flow through the vessel being treated. Any atheroembolic debris trapped upstream of the occluder element may be aspirated using a separate catheter, with or without irrigation of the area.

Relative displacement of the protection element ends causes transformation of the protection element between a closed configuration and an expanded configuration that spans the vessel to be treated. An operator rod, which may be a hollow rod or an interventional catheter, may be slidably disposed over the core wire to engage with and either push or pull the proximal end of the protection element to cause transformation thereof.

### BRIEF DESCRIPTION OF THE DRAWINGS

Features, aspects and advantages of the present invention will become better understood with reference to the following description, appended claims, and accompanying drawings where:
FIG. 1 is an illustration of a guide wire apparatus in accordance with the invention deployed within a blood vessel;
FIG. 2 is an illustration of a guide wire apparatus in accordance with the invention deployed within a portion of the coronary arterial anatomy;
FIG. 3 is an illustration of a prior art expandable mesh device, shown with the mesh in a collapsed configuration;
FIG. 4 is an illustration of a prior art expandable mesh device, shown with the mesh in a deployed configuration;
FIG. 5 is a longitudinal sectional view of a first embodiment in accordance with the invention;
FIG. 6 is a longitudinal sectional view of a second embodiment of a protection element for use with a guide wire apparatus in accordance with the invention, shown in a collapsed configuration;
FIGS. 7 is a longitudinal sectional view of the second embodiment of a protection element for use with a guide wire apparatus in accordance with the invention, shown in an expanded configuration;
FIG. 8 is an elevation view of the second embodiment in accordance with the invention, shown in a collapsed configuration on a guide wire; and
FIG. 9 is an elevation view of the second embodiment in accordance with the invention, shown in an expanded configuration on a guide wire.

### DETAILED DESCRIPTION OF THE INVENTION

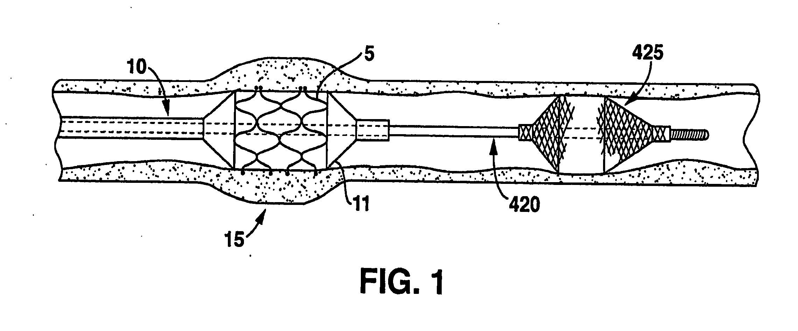

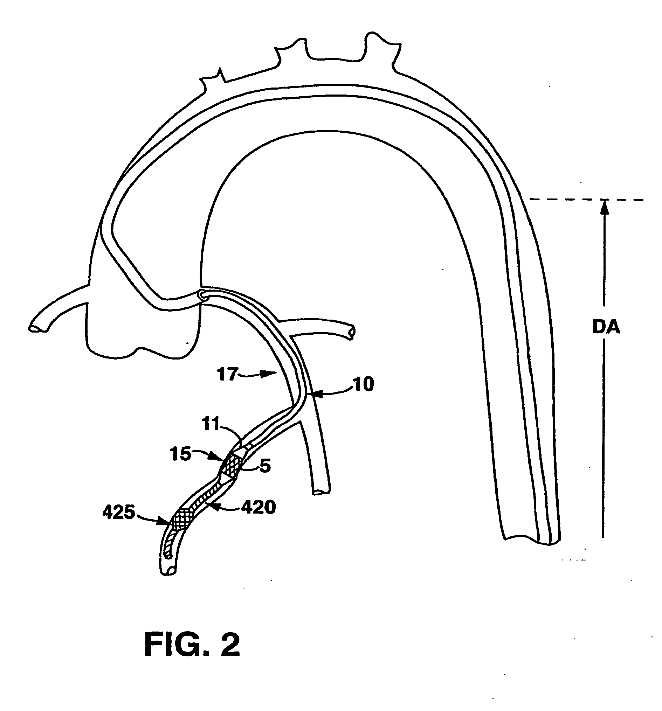

The present invention is a guide wire apparatus for use in minimally invasive procedures. While the following description of the invention relates to vascular interventions, it is to be understood that the invention is applicable to other procedures where the practitioner desires to capture embolic material that may be dislodged during the procedure. Intravascular procedures such as PTCA or stent deployment are often preferable to more invasive surgical techniques in the treatment of vascular narrowings, called stenoses or lesions. With reference to FIGS. 1 and 2, deployment of balloon expandable stent 5 is accomplished by threading catheter 10 through the vascular system of the patient until stent 5 is located within a stenosis at predetermined treatment site 15. Once positioned, balloon 11 of catheter 10 is inflated to expand stent 5 against the vascular wall to maintain the opening. Stent deployment can be performed following treatments such as angioplasty, or during initial balloon dilation of the treatment site, which is referred to as primary or direct stenting.

Catheter 10 is typically guided to treatment site 15 by a guide wire. In cases where the target stenosis is located in tortuous vessels that are remote from the vascular access point, such as coronary arteries 17 shown in FIG. 2, a steerable guide wire is commonly used. According to the present invention, a guide wire apparatus generally guides catheter 10 to treatment site 15 and includes a distally disposed protection element to collect atheroembolic debris that may be generated during the procedure. Various embodiments of the invention will be described as either filter guide wires or occluder guide wires. However, it is to be understood that filters and occluders are interchangeable types of protection elements among the inventive structures disclosed. The invention is directed to atheroembolic protection elements wherein relative movement of the ends of the protection element either causes or accompanies transformation of the element between a collapsed configuration and an expanded, or deployed configuration. Such transformation may be impelled by external mechanical means or by self-shaping memory (either self expanding or self-collapsing) within the protection element itself. The protection element may be self-expanding, meaning that it has a mechanical memory to return to the expanded, or deployed configuration. Such mechanical memory can be imparted to the metal comprising the element by thermal treatment to achieve a spring temper in stainless steel, for example, or to set a shape memory in a susceptible metal alloy such as a nickel-titanium (nitinol) alloy.

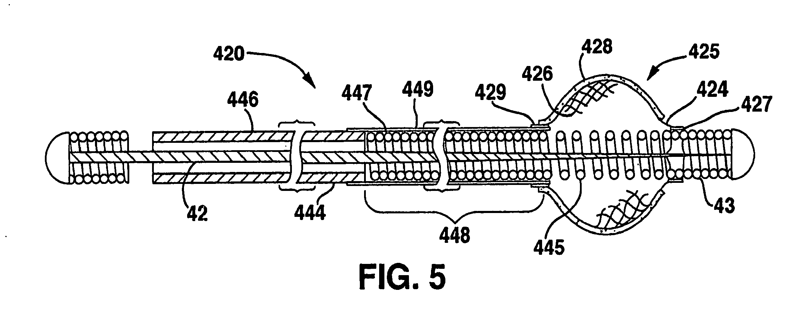

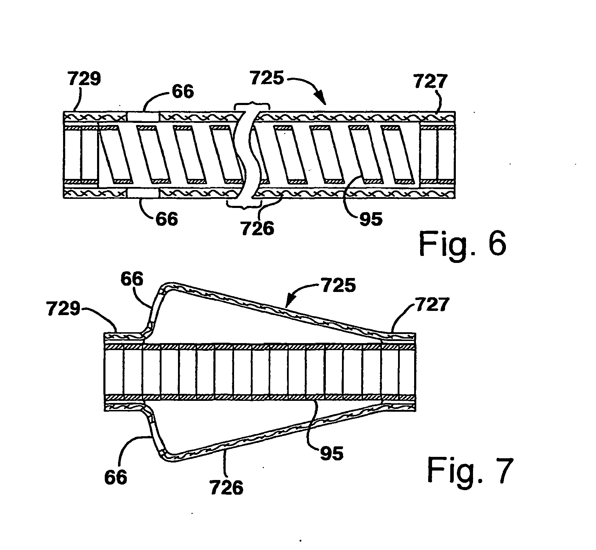

A guide wire apparatus in accordance with the invention includes a distally disposed protection element, such as filter 725, shown in FIG. 6-9, or occluder 425, shown in FIG. 5. Such protection elements may comprise a tube formed by braided filaments 426, which can form a porous filter. Alternatively, braided filaments 426 may be replaced with expandable struts, and either type of support structure can be covered with an elastic membrane. A porous membrane can be used to make a filter element, and non-porous membrane can be used to make an occluder. Optionally, adding radiopaque markers on filter ends can aid in fluoroscopic observation of a protection element during manipulation thereof. Alternatively, to enhance visualization under fluoroscopy, at least one of braided filaments 426 may be a wire having enhanced radiopacity compared to conventional non-radiopaque wires suitable for braiding a protection element. At least the majority of braiding wires forming a protection element should be capable of being heat set into the desired shape, and such wires should also have sufficient elastic properties to provide the desired self-expanding or self-collapsing features. Stainless steel and nitinol monofilaments are suitable for braiding a protection element. A braiding wire having enhanced radiopacity may be made of, or coated with, a radiopaque metal such as gold, platinum, tungsten, alloys thereof, or other biocompatible metals that, compared with stainless steel or nitinol, have a relatively high X-ray attenuation coefficient. One or more filaments having enhanced radiopacity may be inter-woven with non-radiopaque wires, or all wires comprising a protection element may have the same enhanced radiopacity.

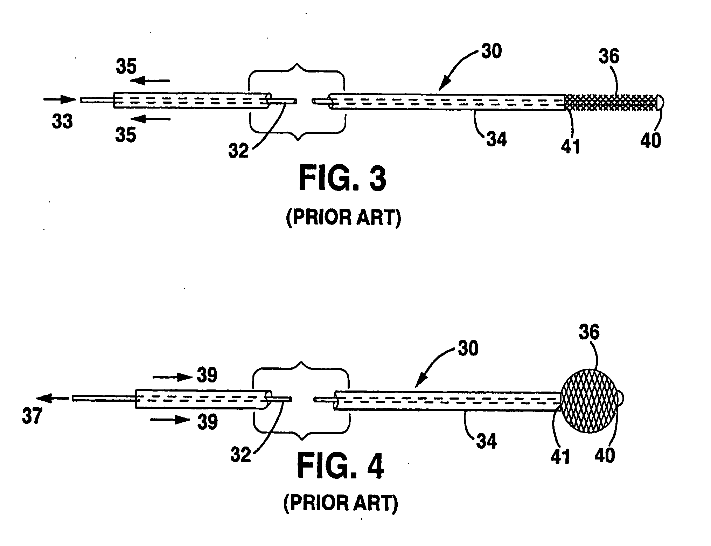

During introduction and withdrawal of a guide wire apparatus of the instant invention, the collapsed protection element does not require a control sheath to slidingly envelope the protection element. Thus, this type of device is sometimes termed "sheathless." Known types of sheathless vascular protection devices are operated by a push-pull mechanism that is also typical of other expandable braid devices, as shown in FIGS. 3 and 4. Prior art expandable mesh 6 device 30 includes core wire 32 and hollow shaft 34 movably disposed there about. Tubular mesh, or braid 36 surrounds core wire 32 and has a braid distal end fixed to core wire distal end 40 and a braid proximal end fixed to shaft distal end 41. To expand braid 36, core wire 32 is pulled and shaft 34 is pushed, as shown by arrows 37 and 39 respectively in FIG. 4. The relative displacement of core wire 32 and shaft 34 moves the ends of braid 36 towards each other, forcing the middle region of braid 36 to expand. To collapse braid 36, core wire 32 is pushed and shaft 34 is pulled, as shown by arrows 33 and 35 respectively in FIG. 3. This reverse manipulation draws the ends of braid 36 apart, pulling the middle region of braid 36 radially inward toward core wire 32.

Referring now to FIG. 5, in a first embodiment of the invention, occluder guide wire 420 includes core wire 42 and flexible tubular tip member 43, such as a coil spring, fixed around the distal end of core wire 42. Thin wires made from stainless steel and/or one of various alloys of platinum are commonly used to make coil springs for such use in guide wires. Core wire 42 can be made from shape memory metal such as nitinol, or a stainless steel wire, and is typically tapered at its distal end. The proximal end of core wire 42 may also be surrounded by a fully bonded coil, which can act as a handle and can help to prevent accidental kinking of the enclosed portion of core wire 42. Tubular shaft 444 is slidably disposed around core wire 42, and includes shaft proximal portion 446 and shaft distal portion 448. Proximal portion 446 is relatively stiff and can be made from thin walled stainless steel tubing, usually referred to as hypotubing, although other metals or rigid polymeric materials can be used. Distal portion 448 is relatively flexible and comprises coiled filament 447 surrounded by sleeve 449. Coiled filament 447 may be made from a plastic filament or metal wire, and its coils are closed, or "stacked" to enhance direct transmission of push force. Sleeve 449 may be made of thin-walled heat-shrink tubing, such as polyethylene terephthalate that has been extruded and blow-molded, or stretch blow-molded, to a larger diameter. Sleeve 449 may be heat-shrunk around coiled filament 447, and optionally, around the distal end of proximal shaft portion 446 to form a joint between proximal and distal portions 446, 448.

The length of shaft distal portion 448 may be selected as appropriate for the intended use of the occluder guide wire. In one example, distal portion 448 may be designed and intended to be flexible enough to negotiate tortuous coronary arteries, in which case the length of distal portion 448 may be 15 - 35 cm (5.9 -13.8 inches), and preferably at least approximately 25 cm (9.8 inches). When occluder guide wire 420 is designed for use in small vessels, shaft 444 may have an outer diameter of about 0.36 mm (0.014 inch). It is advantageous for shaft distal portion 448 to extend proximally from the region of treatment site 15, out of coronary arteries 17, and over the patient's aortic arch. As can be seen in FIG. 2, such a construction would make it unnecessary for relatively stiff, proximal shaft portion 446 to extend from nearly straight descending aorta DA into the comparatively curved aortic arch. In this way, occluder guide wire 420 can have flexible distal portion 448 disposed within tortuous anatomy, while stiff proximal portion 446 can be disposed within linear anatomy. In comparison to treatment of coronary vessels, adaptations of the invention for treatment of renal arteries may require a relatively shorter flexible portion 448, and versions intended for approaching vessels in the head and neck may require a relatively longer flexible portion 448.

Expandable occluder 425 is positioned concentrically with core wire 42, and is sized such that when it is fully deployed, as shown in FIGS. 1 and 2, the outer perimeter of occluder 425 will contact the inner surface of the vessel wall. The surface contact is preferably maintained around the entire vessel lumen to prevent any atheroemboli from slipping past occluder 425. Preferably, cyanoacrylate adhesive is used to secure occluder distal end 427 to tip member 43, and to secure filter proximal end 429 near the distal end of shaft 444. Suitable cyanoacrylate instant adhesives may be obtained from Loctite Corporation, Rocky Hill, CT, U.S.A., or Dymax Corporation, Torrington, CT, U.S.A. Optionally, radiopaque marker bands (not shown), such as platinum rings, can be incorporated into the adhesive joints securing occluder ends 427, 429 respectively to tip member 43 and shaft 444. Occluder 425 is deployed by advancing, or pushing shaft 444 relative to core wire 42 such that occluder distal and proximal ends 427, 429 are forced toward each other, causing the middle, or central section of filter 425 to expand radially. Occluder 425 is collapsed by withdrawing, or pulling shaft 444 relative to core wire 42 such that occluder distal and proximal ends 427,429 are drawn apart from each other, forcing the middle, or central section of occluder 425 to contract radially.

When occluder 425 transforms between a collapsed and an expanded configuration, its interior volume changes, thus potentially creating vacuum or pressure, which may inhibit the desired shape transformation of the protection element. To permit ingress and egress of fluid to and from the interior of occluder 425, non-porous membrane 428 can be fitted over braided filaments 426 such that opening 424 is formed adjacent occluder distal end 427. During shape transformation of occluder 425, fluid, such as blood can flow through opening 424 and through pores formed in braided filaments 426.

Occluder 425 also comprises open coil 445, which surrounds core wire 42 within occluder 425. Open coil 445 may be an expanded, distal extension of coiled filament 447, or it may be formed separately, of filamentous material that is similar to or different from the material of coiled filament 447. Open coil 445 is designed as a compression spring, to assist in collapsing occluder 425. For example, open coil 445 can have a relaxed length that is longer than the collapsed length of occluder 425. In this case, transforming occluder 425 into its expanded configuration will also shorten and compress open coil 445. When acting as a compression spring, open coil 445 will be retained between the distal end of coiled filament 447 and the proximal end of tip member 43, such that it does not need to be fixedly coupled at its ends.

FIGS. 6-9 illustrate self-closing filter 725, a further embodiment of the invention. Filter 725 comprises a tube formed by braided filaments726 that define pores, and at least one inlet opening 66 that is substantially larger than the pores. An alternative filter structure may employ braided filaments 726 to provide support for a porous membrane, in which case the size of the filter pores is defined by the porous membrane rather than by braid filaments 726. Other types of assemblies that may be used in filter 725 include an expandable strut structure (not shown), which may be made from a slotted or slit tube, covered with either a non-porous membrane (not shown) to provide an occluder, or a porous membrane to provide a filter.

Incorporated into filter 725 is spring 95, which is a coiled compression spring disposed within filter 725 and having a distal end affixed to filter distal end 727 and having a proximal end affixed to filter proximal end 729. Spring 95 assists in the collapse of filter 725 by providing separating force between filter distal and proximal ends 727, 729. Assist spring 95 can be fabricated with flat wire, or ribbon, as shown in FIGS. 6 and 7, or with fine metal wire of about 0.03 to 0.13 mm (0.001 to 0.005 inch) diameter, preferably nitinol wire having 0.08 mm (0.003 inch) diameter.

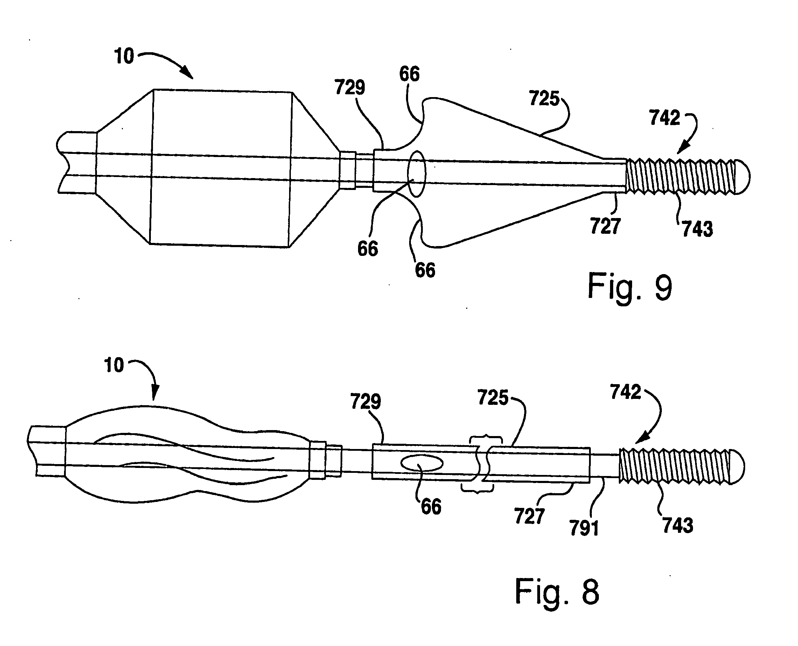

As shown in FIGS. 8 and 9, filter 725 can be selectively loaded onto the proximal end of core wire 742 and slidably advanced there along to a desired location within core wire distal region 791. Filter distal end 727 can be blocked from further distal advancement by a stop element such as tip member 743. In an alternative embodiment, filter 725 can be permanently pre-mounted to core wire 742 by having filter distal end 727 fastened to core wire distal region 791, preferably, with cyanoacrylate adhesive. An elongate operator, such as a hollow push rod or catheter 10 can be advanced over core wire 742 to abut filter proximal end 729 to deploy self-collapsing filter 725. Distally pushing catheter 10, while proximally pulling core wire 742 will transform filter 725 by overcoming the shape memory of filter 725 and/or the compression force in spring 95, as depicted in FIG. 9.

While the invention has been particularly shown and described with reference to the preferred embodiments thereof, it will be understood by those skilled in the art that various changes in form and detail may be made therein without departing from the scope of the invention as defined by the appended claims. For example, the invention may be used in any intravascular treatment utilizing a guide wire where the possibility of loosening emboli may occur. Although the description herein illustrates angioplasty and stent placement procedures as significant applications, it should be understood that the present invention is in no way limited to those environments.

## Claims

1. An apparatus for prevention of distal atheroembolization, the apparatus comprising:
a tubular protection element (425, 725) having distal and proximal ends, wherein relative longitudinal movement between the distal and proximal ends of the protection element (425, 725) accompanies a transformation of the protection element (425, 725) between a closed configuration and an expanded configuration;
**characterized in that** the apparatus further comprises a coiled compression spring (445, 95) disposed within the tubular protection element (425, 725) and having distal and proximal ends coupled to the distal and proximal ends of the protection element (425, 725), respectively, such that the compression spring (445, 95) urges the tubular protection element (425, 725) to remain in the closed configuration, the compression spring (445, 95) being sized and shaped to fit slidably over a core wire (42, 742).

2. The apparatus of claim 1 further comprising a core wire (42, 742) slidably disposed within the protection element (425, 725) and the compression spring (445, 95), the core wire (42, 742) having a distal end and a tip member (43, 743) fixedly mounted adjacent thereto, the tip member (43, 743) limiting distal advancement of the protection element (425, 725) and the compression spring (445, 95) along the core wire (42, 742).

3. The apparatus of claim 2 further comprising an elongate hollow deployment rod slidably and removably disposed about a core wire, the deployment rod being operable to push the protection element (425, 725) distally along the core wire (42, 742) until the protection element (425, 725) is longitudinally compressed between the deployment rod and the tip member (43, 743), thereby effectuating the transformation of the protection element (425, 725) from the closed configuration to the expanded configuration.

4. The apparatus of claim 3 wherein the deployment rod comprises an interventional catheter (10).

5. The apparatus of claim 1 wherein the protection element is a filter (725).

6. The apparatus of claim 1 wherein the protection element is an occluder (425).

## Patentansprüche

1. Vorrichtung zum Verhindern einer distalen Atheroembolisation, wobei die Vorrichtung Folgendes umfasst:
ein röhrenförmiges Schutzelement (425, 725), das ein distales und ein proximales Ende hat, wobei eine relative Längsbewegung zwischen dem distalen und dem proximalen Ende des Schutzelements (425, 725) eine Umformung des Schutzelements (425, 725) zwischen einer geschlossenen Konfiguration und einer expandierten Konfiguration begleitet,
**dadurch gekennzeichnet, dass** die Vorrichtung ferner eine gewundene Druckfeder (445, 95) umfasst, die innerhalb des Schutzelements (425, 725) angeordnet ist und ein distales und ein proximales Ende hat, die an das distale bzw. das proximale Ende des Schutzelements (425, 725) gekoppelt sind derart, dass die Druckfeder (445, 95) das Schutzelement (425, 725) drängt, in der geschlossenen Konfiguration zu verbleiben, wobei die Druckfeder (445, 95) dafür bemessen und geformt ist, verschiebbar über einen Kerndraht (42, 742) zu passen.

2. Vorrichtung nach Anspruch 1, die ferner einen Kerndraht (42, 742) umfasst, der verschiebbar innerhalb des Schutzelements (425, 725) und der Druckfeder (445, 95) angeordnet ist, wobei der Kerndraht (42, 742) ein distales Ende und ein Spitzenelement (43, 743), das unbeweglich an demselben befestigt ist, hat, wobei das Spitzenelement (43, 743) ein distales Vorschieben des Schutzelements (425, 725) und der Druckfeder (445, 95) längs des Kerndrahts (42, 742) begrenzt.

3. Vorrichtung nach Anspruch 2, die ferner eine längliche hohle Entfaltungsstange umfasst, die verschiebbar und abnehmbar um einen Kerndraht angeordnet ist, wobei die Entfaltungsstange betätigt werden kann, um das Schutzelement (425, 725) in Distalrichtung längs des Kerndrahts (42, 742) zu schieben, bis das Schutzelement (425, 725) in Längsrichtung zwischen der Entfaltungsstange und dem Spitzenelement (43, 743) zusammengedrückt wird, wodurch das Umformen des Schutzelements (425, 725) von der geschlossenen Konfiguration zu der expandierten Konfiguration bewirkt wird.

4. Vorrichtung nach Anspruch 3, wobei die Entfaltungsstange einen Interventionskatheter (10) umfasst.

5. Vorrichtung nach Anspruch 1, wobei das Schutzelement ein Filter (725) ist.

6. Vorrichtung nach Anspruch 1, wobei das Schutzelement eine Verschlussvorrichtung (425) ist.

## Revendications

1. Appareil pour empêcher la formation d'athéro-embolie distale, l'appareil comprenant :
un élément de protection tubulaire (425, 725) ayant une extrémité distale et une extrémité proximale, dans lequel un mouvement longitudinal relatif entre l'extrémité distale et l'extrémité proximale de l'élément de protection (425, 725) accompagne une transformation de l'élément de protection (425, 725) entre une configuration fermée et une configuration en expansion ;
**caractérisé en ce que** l'appareil comprend encore un ressort de compression à boudin (445, 95) disposé à l'intérieur de l'élément de protection tubulaire (425, 725) et ayant une extrémité distale et une extrémité proximale couplées à l'extrémité distale et à l'extrémité proximale de l'élément de protection (425, 725), respectivement, de telle façon que le ressort de compression (445, 95) force l'élément de protection tubulaire (425, 725) à rester dans la configuration fermée, le ressort de compression (445, 95) ayant une taille et une forme propres à se placer en coulissement sur un fil d'âme (42, 742).

2. Appareil selon la revendication 1, comprenant en outre un fil d'âme (42, 742) disposé en coulissement dans l'élément de protection (425, 725) et le ressort de compression (445, 95), le fil d'âme (42, 742) ayant une extrémité distale et un élément d'embout (43,743) monté de manière fixe adjacent à lui-même, l'élément d'embout (43,743) limitant l'avancement distal de l'élément de protection (425, 725) et du ressort de compression (445, 95) le long du fil d'âme (42, 742).

3. Appareil selon la revendication 2, comprenant en outre une tige de déploiement creuse allongée, disposée en coulissement et de façon amovible autour d'un fil d'âme, la tige de déploiement pouvant être actionnée pour pousser l'élément de protection (425, 725) en direction distale le long du fil d'âme (42, 742) jusqu'à ce que l'élément de protection (425, 725) soit longitudinalement comprimé entre la tige de déploiement et l'élément d'embout (43, 743) effectuant ainsi la transformation de l'élément de protection (425, 725) de la configuration fermée à la configuration en expansion.

4. Appareil selon la revendication 3, dans lequel la tige de déploiement comprend un cathéter d'intervention (10).

5. Appareil selon la revendication 1, dans lequel l'élément de protection est un filtre (725).

6. Appareil selon la revendication 1, dans lequel l'élément de protection est un élément d'occlusion (425).
